# EUROPEAN PATENT APPLICATION

(11) **EP 1 813 825 A1**
(43) Date of publication of application: **01.08.2007**
(21) Application number: 06100858.7
(22) Date of filing: 25.01.2006
(51) Int. Cl.: F16B 15/00, A61B 17/064

(54) **Fixing Nail**

(71) Applicant: Yea Jann Industrial Co., Ltd., Ar-Lian Township Kao hsiung 822 (TW)
(72) Inventor: Tseng, Ming-Yi, 822, Kaoshiung County (TW)
(74) Representative: Viering, Jentschura & Partner

(57) **Abstract**

A fixing nail includes a pair of legs and a bridge interconnecting the common ends of said legs; said legs have a plurality of annular ridges arranged thereon including a flat surface facing the bridge and an angled surface facing the tapered tip so as to ensure good resistance of extraction after the legs introduced into the materials.

## Description

### BACKGROUND OF THE INVENTION

### 1. Field of the Invention

The present invention relates to a fixing nail, particularly to avoid the retreating when introducing into the materials.

### 2. Description of the Related Art

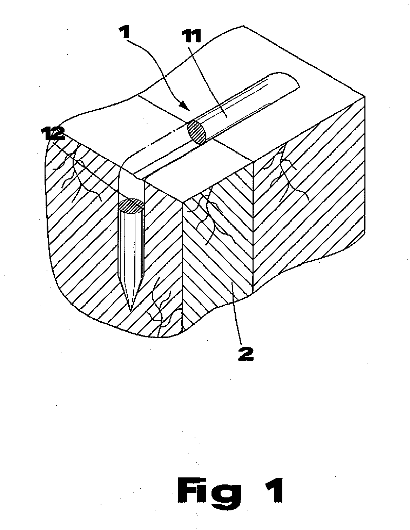

Referring to fig.1, a conventional fixing nail 1 generally includes a pair of spaced-apart longitudinally extending legs 12 and a bridge 11 interconnecting the common ends of two legs 12. Said bridge 11 and legs 12 are smooth-surfaced cylinders. When the fixing nail 1 is pushed in, each leg 12 respectively intrudes into the materials 2 so as to fix two materials 2 together.

In use, the conventional fixing nail 1 has several disadvantages.
1. Due to the fact that the bridge is a smooth-surfaced cylinder, the force is difficultly applied on when pushing the fixing nail into the materials whether by hammer or nail gun.
   Further, the materials can be swung by external force such as vibration force, swing force or any other force that cause movement of two materials. In additional, because said bridge and legs are both smooth-surfaced cylinders, the fixing nail is unable to hold materials tightly through the movement that eventually causes breakaway of said materials.
2. Because said legs are smooth-surfaced cylinders, the friction between said legs and the materials is relatively small that causes the legs easily to extract from the materials after pushing in.
   The present invention intends to provide an improved fixing nail with a plurality of annular ridges arranged on legs which enhance the tightness after clinching the materials.

### SUMMARY OF THE INVENTION

The present invention relates to a fixing nail that comprises a pair of spaced-apart longitudinally extending legs with a tapered tip defined at one common ends thereof, and a bridge interconnecting the other common ends of said legs; said legs have a plurality of annular ridges disposed thereon including a flat surface facing the bridge and an angled surface facing the tapered tip.

The present invention will become more obvious from the following description when taken in connection with the accompanying drawings which show, for purposes of illustration only, a preferred embodiment in accordance with the present invention.

### BRIEF DESCRIPTION OF THE DRAWINGS

Fig. 1 is a perspective view to show the conventional fixing nail pushed in the materials;
Fig. 2 is a perspective view to show the first preferred embodiment of the present invention;
Fig. 3 is a perspective view to show the first preferring embodiment of the present invention, and

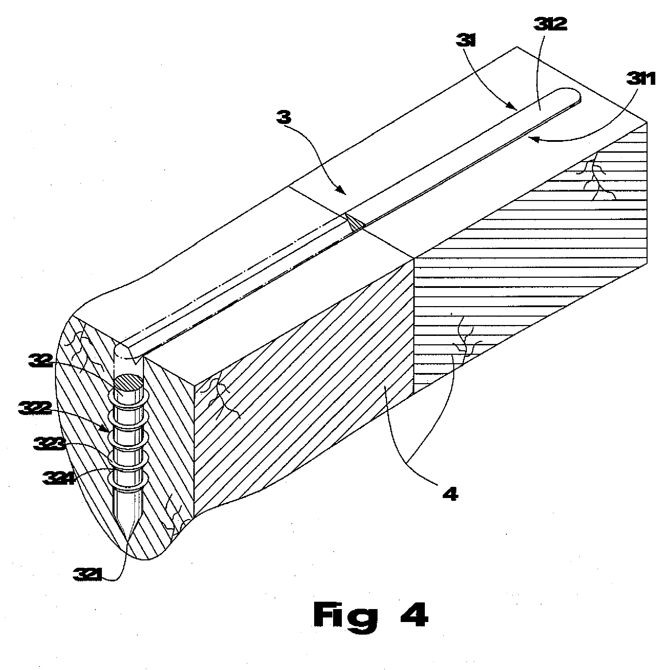

Fig. 4 shows a preferring embodiment of the fixing nail pushed in the materials.

### DETAILED DESCRIPTION OF THE PREFERRED EMBODIMENTS

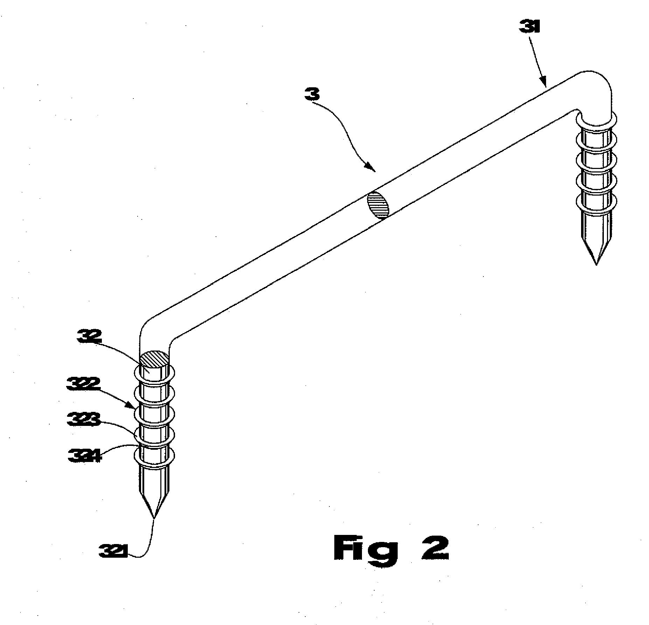

Referring to Fig.2, the fixing nail 3 of the present invention, generally U-shaped, comprises a pair of spaced-apart longitudinally extending legs 32 with a tapered tip 321 defined at one common ends thereof; and a bridge, with a cylindrical configuration, 31 interconnecting the other common ends of said legs 3 2 .Said legs 32 have a plurality of annular ridges 322 disposed thereon including a flat surface 323 facing the bridge 31 and an angled surface 324 facing the tapered tip 321. With the construction of annular ridges 322, said legs are stably engaged with materials which said fixing nail is hardly retreated from materials.

Referring to Fig.2, the second preferring embodiment of present invention of fixing nail 3, wherein said bridge 31 includes a sharp portion 311 defined toward the side that said legs 32 positioned and a flat portion 312 defined on the opposite side of said sharp portion 311 so as the bridge 31 forms a triangular cross-sectional configuration.

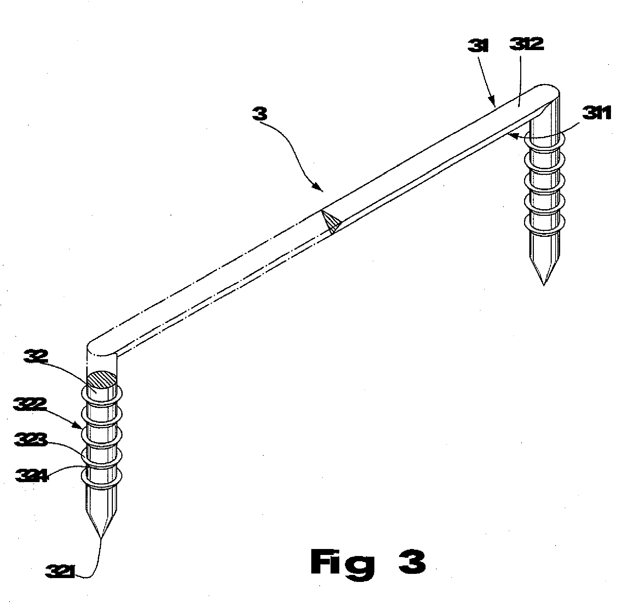

As shown in Fig. 3, during the operation, the users apply a force to the flat portion 312 of the bridge 31 to vertically push the legs 32 into the materials 4. At the same time the legs 32 are completely merged in the materials 4, one groove is defined on the surface of the materials 4 by the cutting of sharp portion 311 so as to accommodate said bridge 31.

As indicated above, the invention has the following advantages:
1. By the means of the legs with annular ridges arranged, it ensures the good resistance of extraction from the materials.
2. Because the sharp portion is located toward the side the legs positioned, a groove is defined on the surface of the materials to accommodate the bridge so as to stabilize and immobilize two materials.
3. By the means of a flat portion on the bridge, it is convenient for users to apply a force to the bridge so as to push the legs into the materials rapidly and avoid the legs from bending.

## Claims

1. A fixing nail comprising:
A pair of spaced-apart longitudinally extending legs with a tapered tip defined at one common ends thereof and a bridge interconnecting the other common ends of said legs; a plurality of annular ridges is arranged on said legs; each of said annular ridges includes horizontally flat surface facing the bridge and an angled surface facing the tapered tip.

2. The fixing nail as claimed in claim1, wherein said bridge has at least one sharp portion defined toward the side that said legs positioned and a flat portion defined on the opposite side of said sharp portion.

3. The fixing nail as claimed in claim1, wherein said bridge has a triangular cross-sectional configuration.
